# EUROPÄISCHE PATENTSCHRIFT

(11) **EP 0 689 532 B1**
(45) Veröffentlichungstag und Bekanntmachung des Hinweises auf die Patenterteilung: **28.05.1997**
(21) Anmeldenummer: 94900800.7
(22) Anmeldetag: 10.11.1993
(51) Int. Cl.: C07C 213/06, C07C 219/06, C07C 219/08, A61K 7/06

(54) **VERFAHREN ZUR HERSTELLUNG VON FESTEN ESTERQUATS MIT VERBESSERTER WASSERDISPERGIERBARKEIT**
METHOD OF PRODUCING SOLID QUATERNARY ESTERS WITH IMPROVED DISPERSIBILITY IN WATER
PROCEDE POUR PREPARER DES ESTERS QUATERNAIRES SOLIDES A DISPERSIBILITE DANS L'EAU AMELIOREE

(30) Priorität: 18.03.1993 DE 4308794
(43) Veröffentlichungstag der Anmeldung: 03.01.1996
(73) Patentinhaber: Henkel Kommanditgesellschaft auf Aktien, 40191 Düsseldorf (DE)
(72) Erfinder: PRAT QUERALT, Ester, E-08728 Barcelona (ES); BIGORRA LLOSAS, Joaquim, E-08203 Sabadell (ES)
(86) Internationale Anmeldenummer: EP9303152
(87) Internationale Veröffentlichungsnummer: WO9421593

(56) Entgegenhaltungen:
- EP-A- 0 008 839
- WO-A-91/01295
- DE-A- 4 138 630

## Beschreibung

Die Erfindung betrifft feste Esterquats mit verbesserter Wasserdispergierbarkeit, die man erhält, indem man Fettsäuretriethanolaminester in Gegenwart von Dispergatoren quaterniert sowie die Verwendung der Stoffe zur Herstellung von Haarpflegemitteln.

### Stand der Technik

Schädigungen der Haarstruktur sind die Folge häufigen Bleichens, Dauerwellens, Färbens, starker UV-Belastung, Waschens der Haare mit entfettenden Tensiden sowie das Ergebnis einer normalen Alterung. Das Haar wird spröde und verliert seinen Glanz. Des weiteren findet beim Kämmen des Haares eine elektrostatische Aufladung statt, während die aufgerauhte Haaroberfläche Anlaß zu Verfilzungen und Verknotungen des Haares gibt und auf diese Weise das Kämmen erschwert. Haarpflegemittel mit einer kämmbarkeitsverbessernden Wirkung haben daher erhebliche Bedeutung auf dem Kosmetikmarkt erlangt. Derartige Mittel können beispielsweise in Form einer Spülung, eines Aerosol-Schaums oder auch in Form von Emulsionen (z.B. Creme-Rinses) nach der Haarwäsche im noch nassen Haar verteilt und entweder nach einigen Minuten Einwirkungszeit ausgespült oder auf dem Haar belassen werden.

Als Wirkstoffe zur Verbesserung der Haarstruktur haben sich kationische Tenside, insbesondere quaternäre Ammoniumverbindungen wie beispielsweise Distearyldimethylammoniumchlorid (DSDMAC) alleine oder in Kombination mit verschiedenen wachsartigen Zusätzen, wie Kohlenwasserstoffen, Fettalkoholen oder Fettsäureestern bewährt [**Parf.Kosm. 56, 157 (1975)**]. Von Nachteil ist hierbei jedoch, daß die genannten Kationtenside eine unzureichende biologische Abbaubarkeit aufweisen und somit bei Eintragung in Oberflächengewässer im Laufe der Zeit die Funktionsfähigkeit aquatischer Lebensgemeinschaften beeinträchtigen können.

Aus der Deutschen Patentanmeldung **DE-A1 35 27 974** sind darüber hinaus Ester des Betains mit Fettalkoholen oder Fettalkoholpolyglycolethern für den Einsatz in sauren Haarpflegemitteln bekannt. Die Betainester weisen zwar eine hohe ökotoxikologische Verträglichkeit auf, sind jedoch im Hinblick auf Kämmbarkeitsverbesserung, Antistatik, Griff und Ausspülverhalten unbefriedigend und zudem im sauren Bereich nicht hydrolysestabil. Gemäß der Lehre der **EP-A 0 008 839** (Akzo) lassen sich tert. Alkylamine in Gegenwart von nichtionischen Phasentransferkatalysatoren wie beispielsweise Sorbitanestern oder Fettalkoholpolyglycolethern quaternieren.

In der älteren Deutschen Patentanmeldung **DE-A1 41 38 630** hat die Anmelderin bereits vorgeschlagen, als kationische Tenside quaternierte Fettsäuretriethanolaminester-Salze, sogenannte "Esterquats", in sauren Haarpflegemitteln einzusetzen. In der Praxis hat sich jedoch gezeigt, daß sowohl die Wasserdispergierbarkeit dieser Produkte, als auch ihre Lagerstabilität nicht immer voll zufriedenstellend ist.

Die Aufgabe der Erfindung hat somit darin bestanden, ein Verfahren zur Herstellung von Esterquats mit verbesserter-Wasserdispergierbarkeit und höherer Lagerbeständigkeit zu entwickeln, die sich mit Vorteil in Haarpflegemitteln einsetzen lassen.

### Beschreibung der Erfindung

Gegenstand der Erfindung ist ein Verfahren zur Herstellung fester Esterquats mit verbesserter Wasserdispergierbarkeit, bei dem man Fettsäuretriethanolaminester der Formel (I), 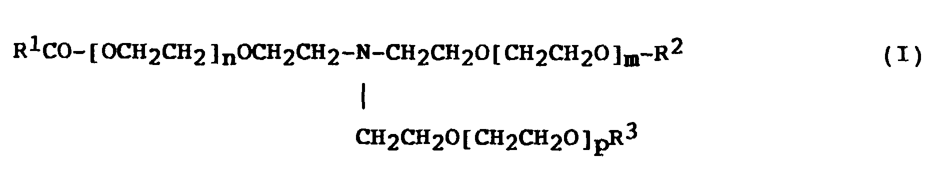 in der R¹CO für einen gesättigten und/oder ungesättigten Acylrest mit 6 bis 22 Kohlenstoffatomen, R² und R³ unabhängig voneinander für Wasserstoff oder R¹CO und n, m und p in Summe für 0 oder Zahlen von 1 bis 10 stehen, in Gegenwart von Dispergatoren, ausgewählt aus der Gruppe, die gebildet wird von
(a) Fettalkoholen und/oder
(b) Dialkylethern
in an sich bekannter Weise mit Alkylierungsmitteln quaterniert.

Überraschenderweise wurde gefunden, daß sich die Quaternierung von Fettsäuretriethanolaminestern auch in Gegenwart der genannten Dispergatoren durchführen läßt. Auf diese Weise werden lösemittelfreie, insbesondere alkoholfreie, feste Esterquats erhalten, die sich leicht in Wasser dispergieren lassen. Die Erfindung schließt die Erkenntnis ein, daß der nachträgliche Zusatz der genannten Dispergatoren zu nach konventionellen Verfahren hergestellten Esterquats die Wasserdispergierbarkeit nicht oder nur sehr geringfügig verbessert. Ein weiterer Vorteil besteht darin, daß die wäßrigen Lösungen der nach dem erfindungsgemäßen Verfahren erhältlichen Esterquats besonders lagerstabil sind, d.h., auch bei längerer Lagerung homogen bleiben und eine konstante Viskosität aufweisen. Produkte, denen schon bei der Quaternierung Dispergatoren zugesetzt worden sind, ergeben nach Auflösen in Wasser unmittelbar ein Haarpflegemittel mit ausgezeichneten anwendungstechnsichen Eigenschaften.

### Esterquats und Fettsäuretriethanolaminester

Esterquats stellen eine bekannte Gruppe kationischer Tenside dar, die üblicherweise durch Veresterung von Triethanolamin bzw. Triethanolaminpolyglycolethern mit Fettsäuren und nachfolgende Quaternierung in organischen Lösungsmitteln erhalten werden. Herstellung und Eigenschaften der Esterquats sind beispielsweise in der **WO 91/01 295** (Henkel) sowie den Übersichtsartikeln von O.Ponsati in **C.R. CED-Kongress, Barcelona, 167 (1992)** und R.Puchta in **C.R. CED-Kongress, Sitges, 59 (1993)** beschrieben.

Vorzugsweise werden technische Mono-/Di-/Triester-Gemische eingesetzt, bei denen der Veresterungsgrad im Bereich von 1,2 bis 2,2, vorzugsweise 1,5 bis 1,9 liegt. Besonders bevorzugt sind Ester, die sich von technischen C_{12/18}- bzw. C_{16/18}-Fettsäuren, wie beispielsweise Palmfettsäure, Kokosfettsäure oder Talgfettsäure ableiten und eine Iodzahl im Bereich zwischen 0 und 40 aufweisen können.

### Dispergatoren

Als geeignete Dispergatoren kommen insbesondere **Fettalkohole** der Formel **(II)** in Frage,

**R**^{**4**}**-OH (II)**

in der R⁴ für einen Alkyl- und/oder Alkenylrest mit 12 bis 22 Kohlenstoffatomen steht. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol und Erucylalkohol sowie deren technische Gemische, wie sie beispielsweise bei der Hochdruckhydrierung von Fettsäuremethylestern oder Aldehyden aus der Roelen'schen Oxosynthese anfallen. Vorzugsweise werden technische Kokos- oder Talgfettalkohole mit 12 bis 18, vorzugsweise 16 bis 18 Kohlenstoffatomen eingesetzt.

Als weitere Dispergatoren kommen auch **Dialkylether** der Formel (III) in Betracht,

**R**^{**5**}**-O-R**^{**6**} **(III)**

in der R⁵ und R⁶ unabhängig voneinander für Alkyl- und/oder Alkenylreste mit 6 bis 22 Kohlenstoffatomen stehen. Auch hierbei handelt es sich um bekannte Stoffe, die nach den einschlägigen Verfahren der präparativen organischen Chemie erhalten werden können. Verfahren zu ihrer Herstellung, beispielsweise durch Kondensation von Fettalkoholen in Gegenwart von p-Toluolsulfonsäure, sind beispielsweise aus **Bull.Soc. Chim.France**, 333 (1949), **DE-A1 40 39 950** (Hoechst) sowie **DE-A1 41 03 489** (Henkel) bekannt. Aus anwendungstechnischer Sicht sind symmetrische Dialkylether bevorzugt, die 6 bis 12 Kohlenstoffatomen in den Alkylresten aufweisen. Als besonders vorteilhaft haben sich Dialkylether der Formel **(III)** erwiesen, in der R⁵ und R⁶ für Octyl- und/oder 2-Ethylhexylreste stehen. Die im Sinne der Erfindung besonders bevorzugten Dialkylether sind somit Di-n-octylether und Di-2-ethylhexylether.

### Emulgatoren

Die Quaternierung kann zusätzlich in Gegenwart von Emulgatoren durchgeführt werden. Als Emulgatoren kommen beispielsweise **Fettalkoholpolyglycolether** der Formel **(IV)** in Betracht,

**R**^{**7**}**O-(CH**_{**2**}**CH**_{**2**}**O)**_{**q**}**H (IV)**

in der R⁷ für einen Alkylrest mit 12 bis 22 Kohlenstoffatomen und q für Zahlen von 10 bis 40 steht. Typische Beispiele sind Anlagerungsprodukte von durchschnittlich 10 bis 40, vorzugsweise 20 bis 25 Mol Ethylenoxid an Fettalkohole mit 12 bis 22 und vorzugsweise 16 bis 18 Kohlenstoffatomen, wie etwa Cetylstearylalkohol-20 EO-Addukt oder Isostearylalkohol-25 EO-Ad- dukt, die eine konventionelle oder eingeengte Homologenverteilung aufweisen können.

Als weitere Emulgatoren können **Anlagerungsprodukte von durch schnittlich 10 bis 40 Mol Ethylenoxid an Fettsäuremonoglyce**ride der Formel (V) eingesetzt werden, 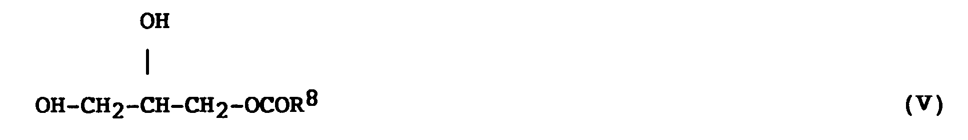 in der R⁸CO für einen Acylrest mit 12 bis 18 Kohlenstoffatomen steht. Typische Beispiele sind Anlagerungsprodukte von durchschnittlich 10 bis 40, vorzugsweise 20 bis 25 Mol Ethylenoxid an technisches Laurinsäuremonoglycerid, Palmitinsäuremonoglycerid, Stearinsäuremonoglycerid und/oder Ölsäuremonoglycerid.

Des weiteren geeignet sind **Fettsäureoligoglyceridpolyglycolether**, d. h. Anlagerungsprodukte von durchschnittlich 10 bis 40, vorzugsweise 20 bis 25 Mol Ethylenoxid an Ester von Fettsäuren mit 12 bis 18 Kohlenstoffatomen mit technischen Oligoglyceringemischen. Typische Beispiele sind Addukte des Ethylenoxids an Mono-, Di-, Tri-, Tetra- und/oder Pentaester des Di-, Tri-, Tetra-, Penta- und/oder Oligoglycerins mit Laurinsäure, Palmitinsäure, Stearinsäure und/oder Ölsäure.

Weiterhin Betracht kommen **Polysorbate**, d. h. Anlagerungsprodukte von durchschnittlich 10 bis 40 Mol Ethylenoxid an Sorbitanester in Frage, deren Fettsäurekomponente sich von Laurinsäure, Palmitinsäure, Stearinsäure und/oder Ölsäure ableitet. Typische Beispiele sind Addukte von durchschnittlich 10 bis 40, vorzugsweise 20 bis 25 Mol Ethylenoxid an Mono-, Di-, Sesqui- und/oder Triester des Sorbitans mit Laurinsäure, Pal- mitinsäure, Stearinsäure und/oder Ölsäure.

Als weitere Emulgatoren können schließlich **Alkyloligoglucoside** der Formel **(VI)** eingesetzt werden,

**R**^{**9**}**O-[G]**_{**z**} **(VI)**

in der R⁹ für einen Alkylrest mit 6 bis 22 Kohlenstoffatomen, G für einen Zuckerrest mit 5 oder 6 Kohlenstoffatomen und z für Zahlen von 1 bis 10 steht. Typische Beispiele sind Alkyloligoglucoside der Formel **(VII)**, in der R⁹ für einen Alkylrest mit 8 bis 18, vorzugsweise 12 bis 14 Kohlenstoffatomen und z für Zahlen von 1,1 bis 1,7 steht.

Wie zuvor schon erläutert, besteht der Kern der Erfindung darin, ein leicht dispergierbares Esterquat zu erzeugen, dem der Dispergator und gegebenenfalls zusätzlich ein Emulgator schon während der Herstellung zugesetzt wird. Abgesehen von den bereits zuvor geschilderten anwendungstechnischen Vorteilen, wird auf diese Weise elegant die ansonsten erforderliche Mitverwendung eines organischen Lösungsmittels in der Quaternierungsstufe umgangen.

Üblicherweise können die erfindungsgemäßen Esterquats die Dispergatoren in solchen Mengen enthalten, daß ihr Anteil 10 bis 90, vorzugsweise 50 bis 70 Gew.-% - bezogen auf das Endprodukt - beträgt. Der Anteil der Emulgatoren kann 0 bis 30, vorzugsweise 5 bis 20 Gew.-% - bezogen auf das Endprodukt-betragen. Praktisch bedeutet dies, daß die Alkylierung in Gegenwart mindestens eines Dispergators durchgeführt wird. Die Erfindung schließt jedoch die Möglichkeit ein, daß die Alkylierung auch in Gegenwart mindestens eines Dispergators und eines Emulgators erfolgt. Für die Einstellung eines gewünschten Dispergator- bzw. Emulgatorgehaltes im Endprodukt ist es erforderlich, die berechnete Menge Dispergator bzw. Emulgator dem Ester vor der Quaternierung zuzusetzen. Die erforderlichen Verhältnisse zu berechnen, bleibt dem Fachmann überlassen, der hierzu nicht erfinderisch tätig werden muß.

### Alkylierung

Die Alkylierung der Fettsäuretriethanolaminester kann in an sich bekannter Weise durchgeführt werden. Hierzu wird der Ester vorgelegt und mit dem Alkylierungsmittel - das man üblicherweise in äquimolaren Mengen oder leichtem Unterschuß einsetzt - bei erhöhten Temperaturen gerührt. Nach Abschluß der Reaktion kann nichtumgesetztes Alkylierungsmittel durch Zugabe einer geringen Menge Aminosäure, vorzugsweise Glycin, zerstört werden. Als Alkylierungsmittel kommen in diesem Zusammenhang Alkylhalogenide, Dialkylsulfate und Ethylenoxid - letzteres in Gegenwart von Dialkylphosphaten - in Betracht. Vorzugsweise betrifft das erfindungsgemäße Verfahren methylquaternierte Esterquats in Form ihrer Chloride oder Methylsulfat-Salze sowie Esterquat-Salze, die mit 1 bis 5 Mol Ethylenoxid quaterniert worden sind.

### Haarpflegemittel

Der Erfindung wird durch Haarpflegemittel illustriert, enthaltend
14 bis 86 Gew.-% quaternierte Fettsäuretriethanolaminester-Salze,
14 bis 86 Gew.-% Dispergatoren
5 bis 30 Gew.-% Emulgatoren,
mit der Maßgabe, daß sich die Prozentangaben zu 100 Gew.-% addieren.

Im Sinne des erfindungsgemäßen Verfahrens werden die Haarpflegemittel unmittelbar erhalten, indem man die Fettsäuretriethanolaminester in Gegenwart der entsprechenden Mengen an Dispergatoren und Emulgatoren quaterniert und das Reaktionsprodukt anschließend in Wasser löst bzw. dispergiert. Es ist jedoch ebenfalls möglich, die festen Esterquats, die nur die Dispergatorkomponente enthalten, in Wasser zu lösen und den Emulgator nachträglich zuzumischen.

Unter Haarpflegemittel sind in diesem Zusammenhang beispielsweise Haarshampoos, Haarspülungen, Haarfestiger, Fönfestiger und dergleichen zu verstehen; vorzugsweise weisen die Mittel einen pH-Wert im Bereich von 3 bis 5, vorzugsweise 3 bis 4 auf.

### Gewerbliche Anwendbarkeit

Die nach denm erfindungsgemäßen Verfahren erhältlichen Produkte sind leicht in Wasser dispergierbar. Die Dispersionen bzw. Emulsionen sind homogen und lagerbeständig und weisen ausgezeichnete anwendungstechnische Eigenschaften im Hinblick auf die Verbesserung der Kämmbarkeit und die Verminderung der elektrostatischen Aufladung von Haaren auf.

Ein weiterer Gegenstand der Erfindung betrifft daher die Verwendung der nach dem erfindungsgemäßen Verfahren erhältlichen festen Esterquats zur Herstellung von Haarpflegemitteln, in denen sie in Mengen von 70 bis 100, vorzugsweise 80 bis 90 Gew.-% - bezogen auf den Feststoffgehalt der Mittel-enthalten sein können.

Die folgenden Beispiele sollen den Gegenstand der Erfindung näher erläutern, ohne ihn darauf einzuschränken.

### Beispiele

### I . Herstellungsbeispiele

a) **Veresterung.** In einem 1-1-Dreihalskolben mit Rührer, Innenthermometer und Destillationsaufsatz wurden 324 g (1,2 mol) teilgehärtete C_{16/18}-Talgfettsäure (Iodzahl = 40), 149 g (1 mol) Triethanolamin und 1,4 g 50 gew.-%ige unterphosphorige Säure gegeben. Über einen Zeitraum von 4 h wurde die Reaktionsmischung bei einem verminderten Druck von 40 mbar auf eine Temperatur von 160°C erhitzt, bis die Säurezahl unterhalb von 5 lag. Anschließend wurde der rohe Talgfettsäuretriethanolaminester abgekühlt, der Reaktionsansatz entspannt und unter ständigem Rühren innerhalb von 15 min 1 Liter Luft durchgeleitet.
b) **Quaternierung.** In einem 500-ml-Dreihalskolben mit Rührer, Tropftrichter und Rückflußkühler wurde eine Mischung von 45 g (0,1 mol) des Esters aus a) in 105 g Dispergator A (C_{16/18}-Talgfettalkohol) bzw. B (Di-n-octylether) vorgelegt und unter Rühren auf 45°C erhitzt (Gewichtsverhältnis Esterquat : Dispergator = 30 : 70). Innerhalb von 2 h wurden 12 g (0,095 mol) Dimethylsulfat zugetropft. Nach Beendigung der Zugabe wurde die Mischung weitere 2 h bei 60°C gerührt und nichtumgesetztes DMS durch Zusatz von 0,4 g (0,005 mol) Glycin zerstört. Das wasserfreie Esterquat/Dispergator-Gemisch wurde als hellfarbige, wachsartige Masse erhalten, die anschließend mechanisch geschuppt wurde.

### II. Anwendungstechnische Beispiele

Die wasserfreien, festen Esterquats aus I) wurden in eine saure Haarspülung der folgenden Rezeptur eingearbeitet:

| | |
|---|---|
| Esterquat | 5,7 Gew.-% |
| Emulgator | 0,5 Gew.-% |
| Wasser | ad 100 Gew.-% |

Als Emulgator wurde Cetylstearylalkohol-20 EO (Eumulgin^{(R)} B2, Fa.Henkel KGaA, Düsseldorf/FRG) eingesetzt; der pH-Wert der Spülungen wurde auf 3,5 eingestellt. Die Emulsionsbildung erfolgte unter schwachem Rühren bei Raumtemperatur. In allen Fällen wurden homogene, kosmetisch elegante Emulsionen erhalten. Die Viskosität der Emulsionen wurde nach 1, 2 und 15 d Lagerung bestimmt (Brookfield RVT, 20°C, 10 Upm). Die Ergebnisse sind in Tab.1 zusammengefaßt:

**Tab.1:**

| Viskositätsmessungen | | | | | |
|---|---|---|---|---|---|
| Bsp. | Esterquat | Dispergator | Viskosität (mPas) | | |
| | | | 1 d | 2 d | 15 d |
| 1 | A1 | A | 9000 | 9400 | 8250 |
| 2 | A1 | B | 9050 | 9350 | 8300 |

Zum Vergleich wurde ein handelsübliches Esterquat (Dehyquart^{(R)} AU 36, 90 Gew.-%ig in Isopropylalkohol, Fa.Pulcra S.A., Barcelona/ES) zunächst vom Lösungsmittel befreit, anschließend - also nachträglich - mit den genannten Dispergatoren A bzw. B vermischt und in die genannte Rahmenrezeptur eingesetzt.

In beiden Vergleichsversuchen zeigte sich, daß zur Emulsionsbildung eine im Vergleich deutlich höhere Scherleistung erforderlich war. Im Hinblick auf die Viskosität der Emulsionen wurden zwar ähnliche Anfangswerte erreicht, jedoch schon nach kurzer Lagerung eine rasche Viskositätsabnahme beobachtet.

## Patentansprüche

1. Verfahren zur Herstellung fester Esterquats mit verbesserter Wasserdispergierbarkeit, bei dem man Fettsäuretriethanolaminester der Formel **(I)**, 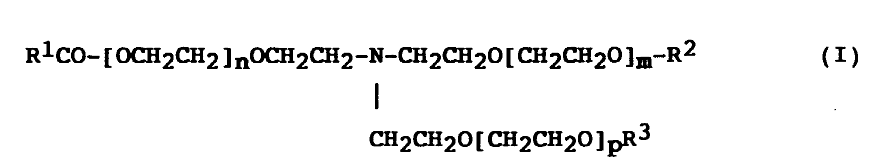 in der R¹CO für einen gesättigten und/oder ungesättigten Acylrest mit 6 bis 22 Kohlenstoffatomen, R² und R³ unabhängig voneinander für Wasserstoff oder R¹CO und n, m und p in Summe für 0 oder Zahlen von 1 bis 10 stehen, in Gegenwart von Dispergatoren, ausgewählt aus der Gruppe, die gebildet wird von
(a) Fettalkoholen und/oder
(b) Dialkylethern
in an sich bekannter Weise mit Alkylierungsmitteln quaterniert.

2. Verfahren nach Anspruch 1, **dadurch gekennzeichnet**, daß man als Dispergatoren Fettalkohole der Formel (II) einsetzt,
**R**^{**4**}**-OH (II)**
in der R⁴ für einen Alkyl- und/oder Alkenylrest mit 12 bis 22 Kohlenstoffatomen steht.

3. Verfahren nach den Ansprüchen 1 und 2, **dadurch gekennzeichnet**, daß man als Dispergatoren Dialkylether der Formel (III) einsetzt,
**R**^{**5**}**-O-R**^{**6**} **(IV)**
in der R⁵ und R⁶ unabhängig voneinander für Alkyl- und/ oder Alkenylreste mit 6 bis 22 Kohlenstoffatomen stehen.

4. Verfahren nach den Ansprüchen 1 bis 3, **dadurch gekennzeichnet**, daß man die Dispergatoren in einer solchen Menge eingesetzt werden, daß ihr Anteil am Endprodukt 10 bis 80 Gew.-% beträgt.

5. Verwendung von festen Esterquats mit verbesserter Wasserdispergierbarkeit nach dem Verfahren nach den Ansprüchen 1 bis 4 zur Herstellung von Haarpflegemitteln.

## Claims

1. A process for the production of solid esterquats with improved dispersibility in water, in which fatty acid triethanolamine esters corresponding to formula (I): 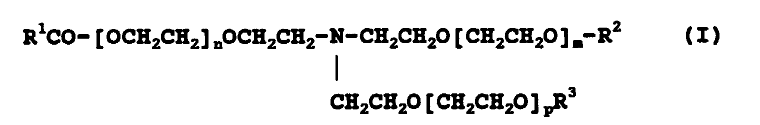 in which R¹CO is a saturated and/or unsaturated acyl radical containing 6 to 22 carbon atoms, R² and R³ independently of one another represent hydrogen or have the same meaning as R¹CO and n, m and p together have a value of 0 or 1 to 10,
are quaternized with alkylating agents in known manner in the presence of dispersants selected from the group consisting of
(a) fatty alcohols and/or
(b) dialkyl ethers.

2. A process as claimed in claim 1, characterized in that fatty alcohols corresponding to formula (II):
**R**^{**4**}**-OH (II)**
in which R⁴ is an alkyl and/or alkenyl radical containing 12 to 22 carbon atoms,
are used as the dispersants.

3. A process as claimed in claims 1 and 2, **characterised in that** dialkyl ethers corresponding to formula (III):
**R**^{**5**}**-O-R**^{**6**} **(IV)**
in which R⁵ and R⁶ independently of one another represent alkyl and/or alkenyl radicals containing 6 to 22 carbon alkyl and/or alkenyl radicals containing 6 to 22 carbon atoms,
are used as the dispersants.

4. A process as claimed in claims 1 to 3, **characterized in that** the dispersants are used in such a quantity that they make up from 10 to 80% by weight of the end product.

5. The use of the solid esterquats with improved dispersibility in water produced by the process claimed in claims 1 to 4 for the production of hair-care formulations.

## Revendications

1. Procédé de préparation d'esters quaternaires solides aptes à la dispersion dans l'eau améliorée où on quaternise un ester d'acide gras et de triéthanolamine de formule (I), 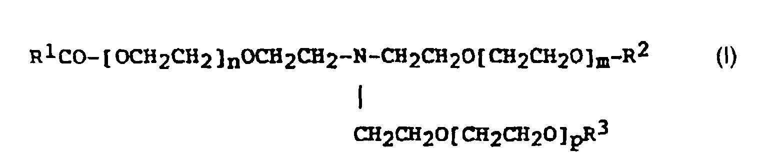 où R¹CO est un radical acyle saturé et/ou insaturé de 6 à 22 atomes de carbone, R et R représentent indépendamment l'un de l'autre l'hydrogène ou R¹CO et n, m et p représentent au total 0 ou des nombres de 1 à 10, en présence de dispersants choisis dans le groupe qui est formé par,
a) des alcools gras et/ou
b) les éthers dialkyliques
de la manière déjà connue avec des agents d'alkylation.

2. Procédé selon la revendication 1,
caractérisé en ce qu'on utilise comme dispersants des alcools gras de formule (II),
R⁴-OH (II)
où R⁴ est un radical alkyle et/ou alcényle avec 12 à 22 atomes de carbone.

3. Procédé selon les revendications 1 et 2,
caractérisé en ce qu'
on utilise comme dispersants des éthers dialkyliques de formule (III),
R⁵-O-R⁶ (III)
où R⁵ et R⁶ représentent indépendamment l'un de l'autre des radicaux alkyles et/ou alcényles de 6 à 22 atomes de carbone.

4. Procédé selon les revendications 1 à 3,
caractérisé en ce qu'
on utilise les dispersants en une quantité telle que leur proportion dans le produit final représente 10 à 80 % en poids.

5. Utilisation d'esters quaternaires solides aptes à la dispersion dans l'eau améliorée selon le procédé des revendications 1 à 4 pour produire des produits de soins capillaires.
